**Europäisches Patentamt**

**European Patent Office**

**Office européen des brevets**

(19)

(11) Veröffentlichungsnummer: **0 023 003**
**B1**

(12) **EUROPÄISCHE PATENTSCHRIFT**

(45) Veröffentlichungstag der Patentschrift:
**02.11.83**

(51) Int. Cl.³: **F 16 M 11/12, G 02 B 7/00**

(21) Anmeldenummer: **80104049.4**

(22) Anmeldetag: **14.07.80**

(54) **Anordnung an einer Tragvorrichtung für ein optisches Beobachtungsgerät.**

(30) Priorität: **24.07.79 CH 6848/79**

(43) Veröffentlichungstag der Anmeldung:
**28.01.81 Patentblatt 81/4**

(45) Bekanntmachung des Hinweises auf die Patenterteilung:
**02.11.83 Patentblatt 83/44**

(84) Benannte Vertragsstaaten:
**AT BE CH DE FR GB IT LI NL SE**

(56) Entgegenhaltungen:
**CH - A - 526 069**
**CH - A - 548 568**
**US - A - 3 475 075**

(73) Patentinhaber: **CONTRAVES AG,**
**Schaffhauserstrasse 580, CH-8052 Zürich (CH)**

(72) Erfinder: **Heller, Rudolf, Köschenrütistrasse 12,**
**CH-8052 Zürich (CH)**
Erfinder: **Schindler, Walter, Carl-Spitteler-Strasse 53,**
**CH-8053 Zürich (CH)**

(74) Vertreter: **Althoff, Gerhard et al, Patentanwälte**
**H.Mitscherlich, K.Gunschmann Dr.W.Körber,**
**J.Schmidt-Evers Steinsdorfstrasse 10,**
**D-8000 München 22 (DE)**

## Anordnung an einer Tragvorrichtung für ein optisches Beobachtungsgerät

Die Erfindung betrifft eine Anordnung an einer Tragvorrichtung für ein optisches Beobachtungsgerät, bei welcher Tragvorrichtung an einer Standsäule ein aus mehreren Teilstücken gebildetes erstes Schwenksystem schwenkbar und an dem ersten Schwenksystem ein im wesentlichen aus einem Tragarm und mehreren Gliedern gebildetes zweites Schwenksystem drehbar gelagert ist, wobei die Schwenk- und/oder Drehbewegungen der beiden Schwenksysteme durch mindestens ein auf einer Stange verstellbar angeordnetes Gegengewicht sowie durch mehrere Dreh- und Bremslager austarierbar sind.

Aus der CH-A-526 069 ist eine als Stativ ausgebildete Tragvorrichtung der eingangs genannten Art bekannt, welche zur Aufnahme eines optischen Beobachtungsgerätes ausgebildet ist und mit dessen Hilfe das Beobachtungsgerät schnell und genau auf jeden Ortspunkt eines Raumgebietes eingestellt werden kann. Das bekannte Stativ umfasst eine Standsäule, einen an einem Querarm des Stativs gelagerten Schwenkarm, eine parallel dazu angeordnete Schubstange, eine am oberen Ende des Schwenkarms gelagerte Stange mit Gegengewicht, mehrere Dreh- und Bremslager sowie einen am unteren Ende des Schwenkarms gelagerten, aus einzelnen Teilstücken gebildeten Tragarm, wobei das Beobachtungsgerät im vorderen Bereich an einem vertikalen Teilstück des Tragarms frei verstellbar und feststellbar gelagert ist.

Bei einer weiteren als Stativ ausgebildeten und mit mehreren Dreh- und Bremslagern versehenen Tragvorrichtung für ein Operationsmikroskop gemäss der CH-A-548 568 ist es bekannt, an der Standsäule einen um eine Horizontalachse schwenkbaren und als Wippe mit zwei Teilstücken ausgebildeten Schwenkarm anzuordnen, an welchem ein erstes Ausgleichsgewicht verschiebbar und feststellbar angeordnet ist. Im oberen Bereich des Schwenkarms ist ein als Waagebalken ausgebildeter Tragarm vorgesehen, an welchem auf der einen Seite ein zur Aufnahme und Befestigung des Operationsmikroskopes ausgebildetes Gelenkparallelogramm und auf der anderen Seite ein zweites, verschieb- und feststellbares Ausgleichsgewicht angeordnet ist. Die Ausbildung der Tragvorrichtung und Anordnung der Ausgleichsgewichte an dem Schwenk- und Tragarm eignet sich insbesondere für Tragvorrichtungen schwererer Bauart ohne besondere Raumbegrenzung.

Der Erfindung liegt die Aufgabe zugrunde, für eine als Stativ ausgebildete Tragvorrichtung, insbesondere für eine Tragvorrichtung leichterer Bauart, eine raum- und gewichtsparende Anordnung der Ausgleichsgewichte zu schaffen, mittels welcher auf einfache Weise die beiden Schwenksysteme mit dem an dem einen Schwenksystem angehängten Beobachtungsgerät in an sich bekannter Weise in seiner Lage und Orientierung frei stehenbleibend austariert werden können, wobei die Tragvorrichtung so ausgelegt sein soll, dass das Anbringen und Abnehmen des Beobachtungsgerätes optimal vorgenommen werden kann.

Diese Aufgabe wird nach der Erfindung dadurch gelöst, dass

a) an dem einen Ende des aus zwei Teilstücken gebildeten Schwenkarms des ersten Schwenksystems ein um eine Horizontalachse schwenkbar gelagerter Lenkhebel und an dem anderen Ende des Schwenkarms eine Gewichtsscheibe sowie die das Gegengewicht tragende Stange angeordnet ist,

b) der Lenkhebel mittels des als Schubstange wirkenden und im wesentlichen parallel zu dem Schwenkarm angeordneten Teilstücks des ersten Schwenksystems mit der das Gegengewicht tragenden Stange wirkverbunden ist, und

c) das erste Schwenksystem in bezug auf die Standsäule und das zweite Schwenksystem in bezug auf das erste Schwenksystem feststellbar ist.

Unter Beibehaltung der im vorbestimmten, räumlichen Bereich hinsichtlich seiner Lage und Orientierung frei stehenbleibenden Verstellbarkeit des Mikroskops wird mit der erfindungsgemässen Anordnung der Ausgleichsgewichte gegenüber den bekannten Tragvorrichtungen in vorteilhafter Weise erreicht, dass der Aufbau der Tragvorrichtung vereinfacht und raumsparend ist.

Eine weitere Vereinfachung besteht darin, dass durch die erfindungsgemässe Arretierung der beiden Schwenksysteme das am Gelenkparallelogramm angehängte Mikroskop gut zugänglich und ohne besonderen Aufwand und ohne weitere Hilfsmittel auswechselbar ist. Die Erfindung wird nachstehend anhand eines Ausführungsbeispieles in Verbindung mit der Zeichnung näher beschrieben. Es zeigt:

Fig. 1 ein in Ansicht dargestelltes Schaubild eines Stativs mit der erfindungsgemässen Anordnung der Schwenk-, Lenk-, Trag- und Bewegungselemente,

Fig. 2 das Stativ gemäss Fig. 1, bei welchem jedoch im wesentlichen der Schwenkarm an der Standsäule und der Lenkhebel am Tragarm arretiert sind,

Fig. 3 im Ausschnitt und in grösserem Massstab den oberen Teil der Schwenk-, Lenk-, Trag- und Bewegungselemente,

Fig. 4 einen Schnitt gemäss der Linie IV–IV in Fig. 3,

Fig. 5 im Ausschnitt und in vergrösserter Darstellung den am Säulenkörper der Standsäule arretierten Schwenkarm, und

Fig. 6 eine teilweise im Schnitt dargestellte Seitenansicht der Arretierung des Schwenkarmes gemäss Fig. 5.

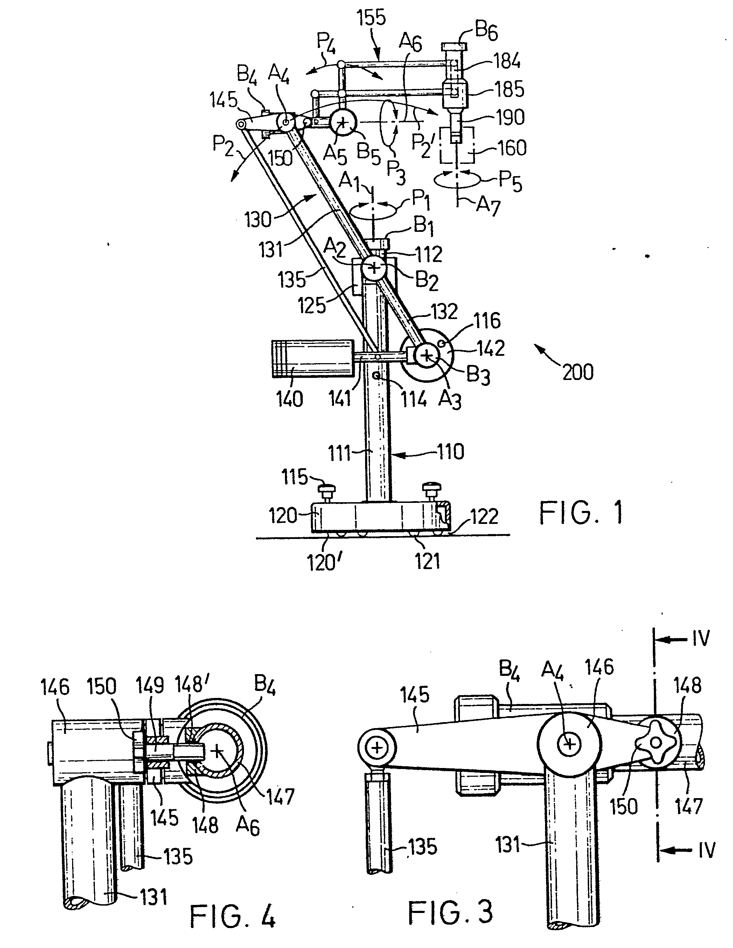

In Fig. 1 ist mit 200 eine mobile, als Bodenstativ ausgebildete Tragvorrichtung für Beobachtungsgeräte, insbesondere für Operationsmikroskope in Ansicht dargestellt, welches im wesentlichen eine Standsäule 110, einen aus zwei Teilstücken 131, 132 gebildeten Schwenkarm 130, einen Tragarm 147 sowie ein aus mehreren Gliedern gebildetes Gelenkparallelogramm 155 aufweist, wobei das Parallelogramm am vorderen Ende zur Aufnahme und Befestigung des schematisch dargestellten Operationsmikroskops 160 ausgebildet ist. Die Standsäule 110 besteht im wesentlichen aus einem auf Rollen 121 gelagerten Stativ-Fuss 120, einem vorzugsweise rohrförmigen Säulenkörper 111 und einem Kopfstück 112 und ist mittels im Fuss 120 angeordneter Schrauben 115 am Boden nivellier- und arretierbar.

Der in Fig. 1 teilweise im Schnitt dargestellte Stativ-Fuss 120 ist vorzugsweise glockenförmig ausgebildet. Die Unterkante 120' des umlaufenden Fussringes reicht zur besseren Standsicherheit des gesamten Stativs 200 bis annähernd zum Boden. Als besonders vorteilhaft hat sich ein Abstand 122 zwischen dem Boden und der Unterkante 120' des Fussringes in der Grössenordnung von 3 bis 5 mm ergeben. Die Rollen 121 sowie die Schrauben 115 sind im Abstand zueinander in bestimmter, nicht dargestellter geometrischer Aufteilung im Fuss 120 angeordnet.

Im oberen Bereich des Säulenkörpers 111 ist das um eine Vertikalachse $A_1$ gegenüber des feststehenden Säulenkörpers in Pfeilrichtung $P_1$ drehbare Kopfstück 112 angeordnet, an welchem seitlich ein Bremslager $B_2$ befestigt ist. An dem Bremslager $B_2$ sind die beiden Teilstücke 131, 132 des Schwenkarmes 130 befestigt. Der Schwenkarm 130 ist einerseits um die Horizontalachse $A_2$ des Bremslagers $B_2$ in Pfeilrichtung $P_2$, $P_2'$ schwenkbar und anderseits zusammen mit dem Kopfstück 112 um die Vertikalachse $A_1$ in Pfeilrichtung $P_1$ drehbar, wobei die Schwenkbewegung $P_2$, $P_2'$ im wesentlichen durch das Bremslager $B_2$ und die Drehbewegung $P_1$ durch ein Bremslager $B_1$ manuell abbremsbar sind.

Fig. 3 und Fig. 4 zeigen in grösserem Massstab den oberen Teil des Stativs 200, und man erkennt das Teilstück 131 des Schwenkarmes sowie den beispielsweise rohrförmigen Tragarm 147. Das Teilstück 131 weist am oberen Ende ein Lager 146 auf, an welchem in nicht näher dargestellter Weise ein um eine Horizontalachse $A_4$ schwenkbar gelagerter Lenkhebel 145 angeordnet ist. An dem Lenkhebel 145 ist auf der einen Seite eine Schubstange 135 angelenkt, und die andere Seite ist zur Lagerung eines griffartig ausgebildeten Arretier-Elementes 150 ausgebildet. Das Elemente 150 weist einen Bolzen, vorzugsweise einen Gewindebolzen 149 auf, welcher in den vorzugsweise mit einer eine entsprechende Öffnung 148' aufweisenden Buchse 148 verstärkten Tragarm 147 einsteck- oder einschraubbar ist. Bei eingeführtem oder eingeschraubtem Element 150 ist der Tragarm 147 mit dem Lenkhebel 145 wirkverbunden. Seitlich am Lager 146 des Lenkhebels 145 ist in nicht näher dargestellter Weise ein Bremslager $B_4$ angeordnet, in welchem in axialer Richtung der Tragarm 147 um eine Längsachse $A_6$ drehbar gelagert ist. Am Ende des Tragarmes 147 ist ein weiteres, quer zum Bremslager $B_4$ angeordnetes Bremslager $B_5$ vorgesehen (Fig. 1, 2).

Wie aus Fig. 1 ersichtlich, ist das aus den gelenkig miteinander verbundenen Gliedern bestehende Parallelogramm 155 mit dem einen Glied am Tragarm 147 und mit dem anderen Glied am Bremslager $B_5$ angelenkt. Die beiden anderen im wesentlichen horizontal orientierten und parallel zueinander verlaufenden Glieder des Parallelogramms weisen an ihren Enden je ein nicht dargestelltes Gelenkstück auf, welche durch einen Steg 184 miteinander verbunden sind. An dem Steg 184 ist ein weiteres Bremslager $B_6$, ein im Gehäuse des Bremslagers drehbar gelagertes Kopfstück 185 sowie ein im Gehäuse des Kopfstückes schwenkbar gelagertes Kupplungsstück 190 befestigt. Das Kupplungsstück 190 ist zur Befestigung des schematisch dargestellten Mikroskops 160 ausgebildet.

Das Parallelogramm 155 mit dem Bremslager $B_6$, den Teilen 184, 185, 190 und 160 ist mittels der am Tragarm angelenkten Glieder um die Drehachse $A_6$ des Tragarmes 147 in Pfeilrichtung $P_3$ drehbar sowie um die quer zur Achse $A_6$ angeordnete Achse $A_5$ in Pfeilrichtung $P_4$ schwenkbar und mittels dem jeweils zugeordneten Bremslager $B_4$ und $B_5$ abbremsbar. Das am Kupplungsstück angehängte Mikroskop ist ausserdem um die Achse $A_7$ in Pfeilrichtung $P_5$ drehbar und durch das Bremslager $B_6$ abbremsbar.

In Fig. 2 ist das Stativ 200 beispielsweise zu Montagezwecken oder zum Auswechseln des in Fig. 2 nicht dargestellten Mikroskops in einer arretierten Stellung dargestellt, und man erkennt den am Säulenkörper 111 arretierten Schwenkarm 130 und durch das Element 150 am Lenkhebel 145 arretierten Tragarm 147 mit dem angelenkten Parallelogramm 155.

Im unteren Bereich des Schwenkarmes 130 ist an dem Teilstück 132 ein Bremslager $B_3$ und eine Gewichtsscheibe 142 angeordnet. Am Gehäuse des Bremslagers $B_3$ ist ausserdem eine Stange 141 zur Aufnahme eines Gegengewichts 140 befestigt. Die das Gegengewicht tragende Stange sowie der am oberen Ende des Schwenkarms 130 angelenkte Lenkhebel 145 sind durch die im wesentlichen parallel zu dem Schwenkarm 130 verlaufende und an der Stange 141 sowie am Lenkhebel 145 angelenkte Schubstange 135 wirkverbunden. Die Schwenkbewegung $P_2$, $P_2'$ des Armes 130 bewirkt eine Schwenkbewegung des Hebels 145 um die Horizontalachse $A_4$ des Lagers 146 sowie der Stange 141 mit dem Gegengewicht um die Horizontalachse $A_3$ des Bremslagers $B_3$, so dass der Lenkhebel 145 mit der Stange 141 und die Schubstange 135 mit dem Schwenkarm 130 in jeder Position weitgehend parallel zueinander verlaufen.

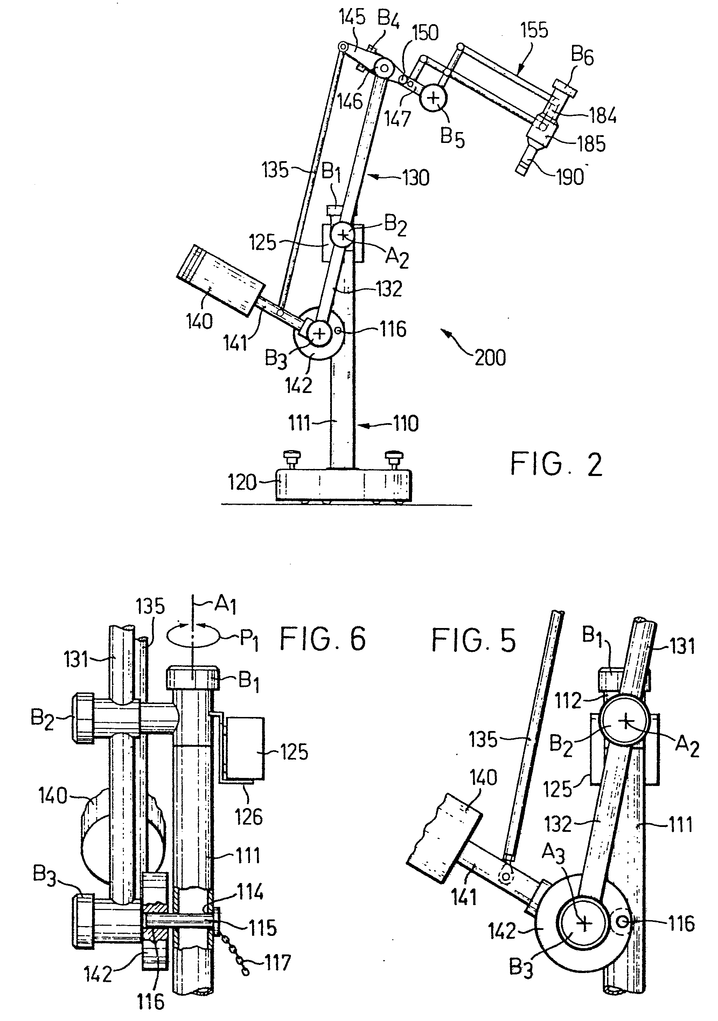

An dem Kopfstück 112 ist am Umfang, versetzt zu dem Bremslager $B_2$, ein Winkelstück 126 befestigt, welches zur Aufnahme eines Instrumentenkastens 125 für die Stromversorgung des Mikroskops dient (Fig. 5, 6).

In Fig. 5 ist in Ansicht und in Fig. 6 in Seitenansicht und teilweise im Schnitt die Arretierung des

5        0 023 003        6

kompletten, aus den Teilstücken 131, 132 gebildeten Schwenkarms 130 an dem Säulenkörper 111 vergrössert dargestellt, und man erkennt das am Teilstück 132 angeordnete Bremslager $B_3$, die Gewichtsscheibe 142, die Stange 141 und das Gegengewicht 140. Das Teilstück 132 mit der Scheibe 142 und dem Gegengewicht 140 ist um die Horizontalachse $A_2$ seitlich wie in Fig. 6 dargestellt an dem Säulenkörper 111 vorbei schwenkbar, wobei diese Schwenkbewegung durch das Bremslager $B_2$ abbremsbar ist. Die Scheibe 142 weist mindestens eine am äusseren Umfang der Scheibe angeordnete Öffnung 116 und der Säulenkörper 111 eine den Körper radial durchdringende Öffnung 114 auf. Sobald bei der Schwenkbewegung die beiden Öffnungen 116, 114 miteinander korrespondieren, kann ein in nicht näher dargestellter Weise beispielsweise mit einer Kette 117 am Säulenkörper befestigter Arretierbolzen 115 durch die Öffnung 114 des Säulenkörpers 111 in die Öffnung 116 der Scheibe 142 eingeschoben werden, wodurch das Teilstück 132 des Schwenkarmes 130 wie in Fig. 6 dargestellt an dem Säulenkörper arretiert ist.

Die Parallel-Bewegung des Lenkhebels 145 zu der Stange 141 mit dem Gegengewicht 140 ist durch das Bremslager $B_3$ einstell- und regulierbar.

Die an den jeweiligen Gelenkstellen des Stativs 200 angeordneten Lager $B_1$, $B_2$, $B_3$, $B_4$, $B_5$ und $B_6$ sind als Dreh- und Bremslager ausgebildet, und jedes einzelne Bremslager ist manuell einstellbar, so dass die auf die Dreh- und/oder Schwenkbewegung der einzelnen Trag-und Schwenkelemente wirkende Bremskraft beliebig den Anforderungen einstellbar ist.

**Patentansprüche**

1. Anordnung an einer Tragvorrichtung (200) für ein optisches Beobachtungsgerät (160), bei welcher Tragvorrichtung an einer Standsäule (110) ein aus mehreren Teilstücken (131, 132 und 135) gebildetes erstes Schwenksystem schwenkbar und an dem ersten Schwenksystem ein im wesentlichen aus einem Tragarm (147) und mehreren Gliedern (155) gebildetes zweites Schwenksystem drehbar gelagert ist, wobei die Schwenk- und/oder Drehbewegungen der beiden Schwenksysteme durch mindestens ein auf einer Stange verstellbar angeordnetes Gegengewicht sowie durch mehrere Dreh- und Bremslager ($B_1$–$B_6$) austarierbar sind, dadurch gekennzeichnet, dass

a) an dem einen Ende des aus zwei Teilstücken (131, 132) gebildeten Schwenkarms (130) des ersten Schwenksystems ein um eine Horizontalachse ($A_4$) schwenkbar gelagerter Lenkhebel (145) und an dem anderen Ende des Schwenkarms (130) eine Gewichtsscheibe (142) sowie die das Gegengewicht (140) tragende Stange (141) angeordnet ist,

b) der Lenkhebel (145) mittels des als Schubstange wirkenden und im wesentlichen parallel zu dem Schwenkarm (130) angeordneten Teilstücks (135) des ersten Schwenksystems mit der das Gegengewicht (140) tragenden Stange (141) wirkverbunden ist, und

c) das erste Schwenksystem in bezug auf die Standsäule (110) und das zweite Schwenksystem in bezug auf das erste Schwenksystem feststellbar ist.

2. Anordnung nach Anspruch 1, dadurch gekennzeichnet, dass der Tragarm (147) des zweiten Schwenksystems an dem Lenkhebel (145) des ersten Schwenksystems sowie der aus den beiden Teilstücken (131, 132) gebildete Schwenkarm (130) mit der Gewichtsscheibe (142) an dem Säulenkörper (111) der Standsäule (110) feststellbar ist.

3. Anordnung nach Anspruch 1 und 2, dadurch gekennzeichnet, dass an dem Lenkhebel (145) ein Arretier-Element (150) und in dem Tragarm (147) eine mit dem Bolzen (149) des Arretier-Elementes (150) korrespondierende Öffnung (148') vorgesehen ist.

4. Anordnung nach Anspruch 1 und 2, dadurch gekennzeichnet, dass in dem Säulenkörper (111) eine den Körper radial durchdringende Öffnung (114) und in der Gewichtsscheibe (142) eine Öffnung (116) vorgesehen ist, und dass die beiden Öffnungen (114, 116) in einer vorbestimmten Stellung bei verschwenktem Schwenkarm (130) miteinander korrespondieren.

5. Anordnung nach Anspruch 2 und 4, dadurch gekennzeichnet, dass der Schwenkarm (130) mit der Gewichtsscheibe (142) mittels eines durch die Öffnung des Säulenkörpers (111) hindurchgesteckten Bolzens (115) an der Standsäule (110) feststellbar ist.

**Claims**

1. Arrangement in a carrier device (200) for an optical observation instrument (160), with which carrier device there is pivotably mounted on an upright (110) a first swivel system formed from several parts (131, 132 and 135) and on the first swivel system there is pivotably mounted a second swivel system formed from a carrier arm (147) and several members (155), whereby the swiveland/or pivoting movements of the two swivel systems can be calibrated by at least one counterweight arranged adjustably on a rod and by several pivot- and brakebearings ($B_1$–$B_6$), characterised in that

a) on the one end of the swivel arm (130) of the first swivel system, formed from two parts (131, 132), there is disposed a steering lever (145) mounted swivellably round a horizontal axis ($A_4$), and on the other end of the swivel arm (130) there is disposed a wheel weight (142) and the rod (141) carrying the counter weight (140),

b) the steering lever (145) is operatively connected to the rod (141) carrying the counter weight (140) by means of the part (135) of the first swivel system working as a driving rod and arranged substantially parallel to the swivel arm (130) and

c) the first swivel system can be secured in position with respect to the upright (110), and the second swivel system with respect to the first swivel system.

2. Arrangement according to claim 1, characterised in that the carrier arm (147) of the second swivel system can be secured in position on the steering lever (145) of the first swivel system, and the swivel arm (130) formed from the two parts (131, 132), with the wheel weight (142), can be secured in position on the upright body (111) of the upright (110).

3. Arrangement according to claim 1 and 2, characterised in that on the steering lever (145) there is provided a locking element (150) and in the carrier arm (147) there is provided an aperture (148') corresponding with the pin (149) of the locking element (150).

4. Arrangement according to claim 1 and 2, characterised in that in the upright body (111) there is provided an aperture (114) radially penetrating the body and in the wheel weight (142) there is provided an aperture (116), and that the two apertures (114, 116) correspond with one another in a predetermined position when the swivel arm (130) is swivelled.

5. Arrangement according to claim 2 and 4, characterised in that the swivel arm (130) with the wheel weight (142) can be secured in position on the upright (110) by means of a pin (115) inserted through the aperture of the upright body (111).

**Revendications**

1. Agencement d'un dispositif de support (200) pour un appareil d'observation optique (160), dans lequel le dispositif de support est monté basculant sur une colonne (110) à l'aide d'un premier système basculant formé de plusieurs pièces (131, 132 et 135), et est monté à rotation à l'aide d'un second système basculant formé essentiellement d'un bras de support (147) et de plusieurs organes (155), les mouvements de basculement et/ou de rotation des deux systèmes basculants permettant de réaliser la tare à l'aide d'au moins un contrepoids prévu de façon réglable sur une tige et de plusieurs paliers de rotation et de paliers-freins (B₁–B₆), agencement caractérisé en ce que:

a) à une extrémité du bras basculant (130) formé de deux pièces (131, 132) du premier système basculant, il est prévu un levier de guidage (145) monté basculant sur un axe horizontal (A₄) et en ce qu'à l'autre extrémité du bras basculant (130), il est prévu un disque pesant (142) ainsi que la tige (141) portant le contrepoids (140),

b) le levier de guidage (145) est relié de façon à coopérer avec la tige (141) portant le contrepoids (140), à l'aide de la pièce (135) fonctionnant comme tige de poussée et qui est essentiellement parallèle au bras basculant (130) dans le premier système basculant,

c) le premier système basculant peut se bloquer par rapport à la colonne (110), et le second système basculant peut se bloquer par rapport au premier système basculant.

2. Agencement selon la revendication 1, caractérisé en ce que le bras de support (147) du second système basculant est susceptible d'être bloqué sur le levier de guidage (145) du premier système basculant ainsi que le bras basculant (130) formé des deux pièces (131, 132) avec le disque pesant (142) sur le corps (111) de la colonne (110).

3. Agencement selon les revendications 1 et 2, caractérisé en ce qu'un élément de blocage (150) est prévu sur le levier de guidage (145) et dans le bras de support (147) il est prévu une ouverture (148') correspondant avec le goujon (149) de l'élément de blocage (150).

4. Agencement selon les revendications 1 et 2, caractérisé en ce que dans le corps de colonne (111), il est prévu une ouverture (114) qui traverse radialement le corps et dans le disque pesant (142), il est prévu une ouverture (116) et en ce que les deux ouvertures (114, 116) se correspondent dans une position prédéterminée lorsque le bras basculant (130) est basculé.

5. Agencement selon les revendications 2 et 4, caractérisé en ce que le bras basculant (130) avec le disque pesant (142) peut se bloquer sur la colonne (110), à l'aide d'un goujon (115) traversant l'orifice du corps de colonne (111).

FIG. 1

FIG. 4

FIG. 3

FIG. 2

FIG. 6

FIG. 5